# EUROPÄISCHE PATENTANMELDUNG

(11) **EP 1 336 599 A1**
(43) Veröffentlichungstag der Anmeldung: **20.08.2003**
(21) Anmeldenummer: 03002103.4
(22) Anmeldetag: 30.01.2003
(51) Int. Cl.: C07C 51/29, C07C 57/20, C07C 57/22, C07C 57/24, C07C 57/42, C07C 67/40, C07C 69/606

(54) **Verfahren zur Herstellung von Alkincarbonsäuren und Alkincarbonsäure-Alkinalkoholestern durch Oxidation von Alkinalkoholen**

(30) Priorität: 15.02.2002 DE 10206619
(71) Anmelder: Consortium für elektrochemische Industrie GmbH, 81379 München (DE)
(72) Erfinder: Stohrer, Jürgen, Dr., 82049 Pullach (DE); Fritz-Langhals, Elke, Dr., 85521 Ottobrunn (DE); Brüninghaus, Christian, 82008 Unterhaching (DE); Stauch, Dagmar, 80802 München (DE)
(74) Vertreter: Schuderer, Michael, Dr.

(57) **Zusammenfassung**

Die Erfindung beschreibt Verfahren zur Herstellung von Alkincarbonsäuren und Alkincarbonsäure-Alkinalkoholestern, gekennzeichnet durch die Oxidation eines Alkinalkohols mit 1 bis 10 mol-Äquivalenten eines Hypohalogenits bezogen auf die Anzahl zu oxidierender funktionaler Gruppen in Gegenwart einer Nitroxylverbindung bei einem pH-Wert kleiner 7 oder gekennzeichnet durch die teilweise Oxidation des Alkinalkohols mit 0,5 bis 5 mol-Äquivalenten eines Hypohalogenits bezogen auf die Anzahl zu oxidierender funktionaler Gruppen in Gegenwart einer Nitroxylverbindung bei einem pH-Wert kleiner 7.

## Beschreibung

Die vorliegende Erfindung beschreibt Verfahren zur Oxidation von Alkinalkoholen (Alkinolen) zu Alkincarbonsäuren (Alkinsäuren) sowie zur Herstellung von Alkincarbonsäure-Alkinalkoholestern.

Alkinsäuren sind wichtige Synthesebausteine. Von besonderer Bedeutung sind Propiolsäure und Acetylendicarbonsäure, die zum Aufbau von Ringen in Cycloadditionen, insbesondere Diels-Alder-Reaktionen und 1,3-dipolaren Cycloadditionen, und bei nucleophilen Additionsreaktionen eingesetzt werden (Übersicht in Ullmann' Encyclopedia, 6^{th} Edition, 2001 electronic release; "Carboxylic acids, aliphatic 5.2").

Die Oxidation von Alkinolen zu Alkinsäuren ist im Stand der Technik beschrieben worden (Übersichten in Ullmann' Encyclopedia,6^{th} Edition, 2001 electronic release; "Carboxylic acids, aliphatic 5.2"; Houben-Weyl Band V/2a, 4. Auflage 1977, "Alkine").

So wird beispielsweise Propiolsäure durch anodische Oxidation von Propargylalkohol erhalten (Wolf, Chem. Ber. 1954, 87, 668). Ebenso erhält man Acetylendicarbonsäure durch anodische Oxidation von 2-Butin-1,4-diol. Das elektrochemische Verfahren hat aber den Nachteil der Verwendung von Bleidioxid-Anoden, die zur Kontamination der Elektrolyten mit Bleiionen führen, und ist generell nur mit grossen Investitionen in der Produktion umsetzbar. Die als Nebenreaktion ablaufende Decarboxylierung des Produkts führt zudem zur technisch unerwünschten Entstehung grosser Mengen an CO₂ und Acetylen, die zu entsorgen sind. Ferner sind die Ausbeuten im Fall der Propiolsäure relativ gering (unter 50%).

Die analoge anodische Oxidation an Nickeloxid-Anoden (Kaulen und Schäfer, Tetrahedron 1982, 38, 3299) erfordert geringe Stromdichten und sehr große Elektrodenoberflächen, was zu einer weiteren Steigerung der Investkosten führt. Zudem wird die aktivierte Nickeloberfläche während der Elektrolyse passiviert und muss häufig regeneriert werden, was zu einer Steigerung der Prozesskosten führt.

Propiolsäure kann außerdem durch Oxidation von Propargylalkohol mit Cr(VI)-Oxid in Schwefelsäure erhalten werden. Hier sind gute Ausbeuten erzielbar, aber grosse Mengen an toxischen und umweltgefährdenden Schwermetallsalzen zu entsorgen. Die analoge Umsetzung von 2-Butin-1,4-diol führt nur mit 23 % zu Acetylendicarbonsäure (Heilbron, Jones und Sondheimer, J. Chem. Soc. 1949, 606).

Als nicht-oxidatives Herstellverfahren von Propiolsäure und Acetylendicarbonsäure ist die Umsetzung von Metall-acetyliden mit CO₂ bekannt. Dies erfordert aber den Einsatz teurer Metallbasen und ist technisch wegen des Einsatzes von Acetylen nicht ohne Gefahr. Die Ausbeuten dieses Verfahrens liegen im Falle von Propiolsäure ebenfalls nur bei 50%.

Bei einem weiteren Verfahren zur Darstellung von Acetylendicarbonsäure wird Fumarsäure mit Brom zunächst in meso-Dibrombernsteinsäure überführt, diese isoliert und in einer weiteren Stufe enthalogeniert. Dieser Zweistufenprozess ist zeitaufwändig und umständlich (T.W. Abbott et al., Org. Synth Coll. Vol. II, 1943, 10).

Allgemeine Oxidationsverfahren von Alkoholen zu Carbonsäuren mit Hilfe von Nitroxylverbindungen als Katalysatoren sind aus dem Stand der Technik bekannt, insbesondere mit Hilfe von TEMPO (2,2,6,6,-Tetramethylpiperidin-1-oxyl) und seinen Derivaten (Übersicht in A.E.J. de Nooy, A.C. Besemer und H.V. Bekkum, Synthesis 1996, 1153).

Zahlreiche TEMPO-Derivate sind als Oxidationskatalysatoren beschrieben, darunter auch TEMPO-Derivate auf polymeren Trägern, beispielsweise PIPO (polyamine immobilised piperidinyl oxyl) (Dijksman et al., Synlett 2001, 102).

Diese TEMPO-katalysierten Oxidationen werden in Zweiphasensystemen, z.B. Methylenchlorid / Wasser, durchgeführt (P.L. Anelli, C. Biffi, F. Montanari und S. Quici, J. Org. Chem. 1987, 52, 2559).

Bei allen diesen Verfahren können aus Alkoholen Carbonsäuren unter zusätzlichem Einsatz von Phasentransferkatalysatoren gewonnen werden (G. Grigoropolulou et al., Chem. Commun. 2001, 547-548, P.L. Anelli, C. Biffi, F. Montanari und S. Quici, J. Org. Chem. 1987, 52, 2559). Als stöchiometrisches Oxidationsmittel wird dabei vorwiegend Bleichlauge (Hypochloritlösung) eingesetzt.

Bei der üblichen Durchführung dieser Synthesen in zweiphasigen Systemen wird das in der Wasserphase gelöste Oxidationsmittel der organischen Phase, die den zu oxidierenden Alkohol und die Nitroxylverbindung enthält, in einem Batch-Verfahren innerhalb eines pH-Bereichs von 9-10 zugesetzt.

Ein großtechnisch durchführbares Verfahren zur Oxidation von ungesättigten Alkinalkoholen (Alkinolen) insbesondere solcher mit terminalen Acetylengruppen zu den entsprechenden Alkincarbonsäuren (Alkinsäuren) mittels Hypochlorit in Gegenwart von Nitroxylverbindungen ist jedoch bislang nicht beschrieben worden.

Eine mögliche Ursache ist die literaturbekannte Empfindlichkeit terminaler Alkingruppen gegenüber Bleichlauge. Die CH-Gruppen terminaler Alkine werden beispielsweise durch Bleichlauge leicht in Chloralkine überführt, die besonders im Alkalischen labil sind und zur Zersetzung neigen (Straus et al., Ber. Dtsch. Chem. Ges. 1930, 1868).

Im Stand der Technik wird beschrieben, dass Oxidationsverfahren unter Verwendung von Bleichlauge und Nitroxylverbindungen für die Oxidation ungesättigter Alkohole generell als ungeeignet zu betrachten sind (vergleiche hierzu insbesondere P.L. Anelli, C. Biffi, F. Montanari und S. Quici, J. Org. Chem. 1987, 52, 2559; P.L. Anelli, F. Montanari und S. Quici, Org. Synth., 1990, 69, 212).

So liefert die Umsetzung eines Alkinalkohols mit nichtterminaler Acetylengruppe mit Bleichlauge und TEMPO nach einem im Stand der Technik bekannten Vefahren nur 5 bis maximal 20 % der Alkinsäure. Die Oxidation gelingt dabei nur unter Verwendung von Natriumchlorit als Oxidationsmittel unter Zusatz katalytischer Mengen Hypochlorit und TEMPO in einem mit Phosphatpuffer bei pH 6-7 gepufferten Zweiphasengemisch (M. Zhao et al., J. Org. Chem. 1999, 64, 2564; WO 99/52849). Bei dieser Methode ist insbesondere die Verwendung des relativ teuren und überdies explosiven Natriumchlorits von Nachteil. Zudem werden große Mengen Phosphatpuffer benötigt, dessen Entsorgung aufwändig und kostspielig ist.

Es bestand daher die Aufgabe, ein Verfahren zur Herstellung von Alkinsäuren und Alkincarbonsäure-Alkinalkoholestern durch die Oxidation von Alkinalkoholen bereitzustellen, dass die aus dem Stand der Technik bekannten Nachteile vermeidet.

Gegenstand der Erfindung ist ein Verfahren zur Herstellung von Alkincarbonsäuren, gekennzeichnet durch die Oxidation eines Alkinalkohols mit 1 bis 10 mol-Äquivalenten eines Hypohalogenits bezogen auf die Anzahl zu oxidierender funktionaler Gruppen in Gegenwart einer Nitroxylverbindung bei einem pH-Wert kleiner 7.

Ein weiterer Gegenstand der Erfindung ist ein Verfahren zur Herstellung von Alkincarbonsäure-Alkinalkoholestern, gekennzeichnet durch die teilweise Oxidation des Alkinalkohols zu Alkincarbonsäure-Alkinalkoholestern mit 0,5 bis 5 mol-Äquivalenten eines Hypohalogenits bezogen auf die Anzahl zu oxidierender funktionaler Gruppen in Gegenwart einer Nitroxylverbindung bei einem pH-Wert kleiner 7.

Verfahrensprodukt ist hierbei der Ester der entsprechenden Alkincarbonsäure und dem nicht umgesetzten Teil des Alkinalkohols.

So wird beispielsweise im Falle von Propargylalkohol als Edukt Propiolsäurepropargylester gebildet.

Generell können in den erfindungsgemäßen Verfahren die an der Reaktion beteiligten Komponenten in einer Phase oder auf mehrere Phasen verteilt umgesetzt werden.

In möglichen Ausführungsformen der erfindungsgemäßen Verfahren wird eine wässrige Phase verwendet oder die Verfahren werden in einer wässrigen Phase durchgeführt.

In einer weiteren möglichen Ausführungsform werden die erfindungsgemäßen Verfahren in einer Phase, die Wasser und ein oder mehrere mit Wasser mischbare Lösungsmittel enthält (Cosolventien), durchgeführt.

Bei einer Ausführungsform der einphasigen Reaktion wird das zu oxidierende Alkinol in reiner Form oder verdünnt mit Wasser oder mit einem oder mehreren inerten mit Wasser mischbaren Solventien zusammen mit der Nitroxylverbindung als Reaktionskomponente 1 vorgelegt und mit dem Oxidationsmittel als Reaktionskomponente 2 versetzt.

In einer alternativen Ausführungsform werden beide Reaktionskomponenten parallel dosiert, wobei zusätzlich das inerte mit Wasser mischbare Solvens oder die mit Wasser mischbaren Solventien oder die Nitroxylverbindung vorgelegt werden können.

Die inerten mit Wasser mischbaren Solventien werden bevorzugt ausgewählt aus der Gruppe der Ether, insbesondere THF und 1,4-Dioxan, der Nitrile, insbesondere Acetonitril oder DMF, DMSO oder tert-Butanol.

Der zu oxidierende Alkinalkohol kann in Konzentrationen zwischen 0,1 und 100 Gew. %, bevorzugt zwischen 20 und 100 Gew. % bezogen auf das zur Verdünnung eingesetzte Wasser oder dem mit Wasser mischbaren Lösungsmittel oder den mit Wasser mischbarten Lösungsmitteln eingesetzt werden.

In einer weiteren möglichen Ausführungsform der erfindungsgemäßen Verfahren werden die Reaktionen in Mehrphasensystemen durchgeführt.

Bevorzugt wird dabei mindestens eine wässrige Phase und eine organische Phase verwendet.

In einer besonders bevorzugten Ausführungsform wird der Alkinalkohol gegebenenfalls in reiner Form oder in einem oder mehreren nicht mit Wasser mischbaren Lösungsmitteln gelöst, eingesetzt.

Wird mehrphasig gearbeitet, so besteht die organische Phase aus dem Alkinalkohol, der Nitroxylverbindung und gegebenenfalls einem oder mehreren organischen Lösungsmitteln. Die zweite wässrige Phase enthält das Hypohalogenit. Die Phasenseparation kann dabei auch ursächlich auf ein nicht mit Wasser mischbaren Alkinalkohol als Edukt oder die Bildung einer nicht mit Wasser mischbaren Alkincarbonsäure oder Alkincarbonsäure-Alkinalkohols als Produkte zurückgehen.

Bevorzugte organische Lösungsmittel zur Durchführung des Verfahrens in einem Mehrphasensystem sind ein oder mehrere Lösungsmittel ausgewählt aus der Gruppe der Ether, insbesondere THF, Methyl-t-Butylether und Diethylether, Acetonitril, Methylenchlorid, Essigsäureethylester, Dimethylsulfoxid (DMSO), tert-Butanol und Toluol.

Der zu oxidierende Alkohol kann in Konzentrationen zwischen 0,1 und 100 Gew. % bezogen auf die organische Phase, bevorzugt zwischen 20 und 100 Gew. % eingesetzt werden.

In einer bevorzugten Ausführungsform des Verfahrens zur Herstellung von Alkincarbonsäure-Alkinalkoholestern wird diese Oxidation in zweiphasigen Reaktionsmischungen unter Verwendung eines oder mehrerer nicht mit Wasser mischbaren Lösungsmittel durchgeführt, wobei der Ester in der organischen Phase anfällt und somit leicht abgetrennt werden kann. Die zweite wässrige Phase enthält das Hypohalogenit. Die gebildeten Ester können durch Behandlung mit wässrigen Laugen in die entsprechenden Alkinsäuren überführt werden, wobei das zugleich anfallende Alkinol rückgewonnen und für weitere Oxidationen eingesetzt werden kann. Alternativ können die Ester auch durch Umesterung in andere Alkinsäureester überführt werden.

Die Vorteile der erfindungsgemäßen Verfahren sind in der Bereitstellung technisch einfacher Verfahren zur Oxidation von Alkinalkoholen zu Alkincarbonsäuren und zur Herstellung von Alkincarbonsäure-Alkinalkoholen unter Verwendung kostengünstiger Hypohalogenite, die die aus dem Stand der Technik bekannten Probleme beheben.

Mit den erfindungsgemäßen Verfahren werden dabei unter Verwendung kostengünstiger und technisch leicht einsetzbarer Bleichlauge (Natriumhypochlorit) selbst gut wasserlösliche Alkinole und Alkinole mit terminalen Alkingruppen in technisch einfacher Weise und guter Ausbeute zu den entsprechenden Alkincarbonsäuren oxidiert.

Die zwingende Einsatz der aus dem Stand der Technik bekannten Zweiphasensysteme zur Oxidation ist insbesondere für solche Substrate nicht mehr notwendig.

Speziell Substrate mit terminalen Alkingruppen waren bislang durch die aus dem Stand der Technik bekannten oxidativen Verfahren nicht durch großtechnisch realisierbare und wirtschaftlich interessante Reaktionen zugänglich.

Darüber hinaus sind die erfindungsgemäßen Verfahren für eine große Breite an Substraten mit terminalen und nicht terminalen Alkingruppen anwendbar.

So kann beispielsweise Propiolsäure mit den erfindungsgemäßen Verfahren aus Propargylalkohol in 45-75 % Ausbeute und Acetylendicarbonsäure aus Butindiol in 50 % Ausbeute erhalten werden.

Die bei der Umsetzung entstehenden Abwässer enthalten nur leicht zu entsorgende Salze wie NaCl und sind daher, im Gegensatz zu Verfahren, bei denen Phosphatpuffer eingesetzt werden müssen, problemlos zu entsorgen.

In einer möglichen Ausführungsform der erfindungsgemäßen Verfahren können die an der Reaktion beteiligten Phasen auch kontinuierlich umgesetzt werden.

Dabei werden die Reaktionskomponenten 1 und 2 kontinuierlich zugeführt und zugleich die entstehende Reaktionslösung kontinuierlich abgezogen. Die Einhaltung des für die Reaktion günstigen pH-Bereiches kann durch eine dritte kontinuierliche Dosierung erfolgen, über die Basen oder Säuren der Reaktionsmischung so zugeführt wird, dass in der Reaktionsmischung ein konstanter pH<7 vorliegt.

Die für ein kontinuierliches Verfahren geeigneten kontinuierlichen Reaktoren sind dem Fachmann bekannt. Eine Übersicht über die wichtigsten Ausführungsformen findet sich beispielsweise in "Ullmann's Encyclopedia of Industrial Chemistry", Vol. B4.

Bevorzugte Ausführungsformen für ein kontinuierlich durchgeführtes Verfahren sind kontinuierlich betriebene Rohrreaktoren, kontinuierlich betriebene Loop-Reaktoren, kontinuierlich betriebene Rührkesseln oder Rührkesselkaskaden oder ein mit Hilfe von Kreiselpumpen durchgeführtes Verfahren.

Wird das beschriebene Verfahren als kontinuierlicher Prozess ausgeführt, erlaubt dies als zusätzlichen Vorteil eine effiziente Wärmeabfuhr aus dem stark exothermen Reaktionsprozess.

Die aus dem Stand der Technik bekannten oxidativen Verfahren waren nur im Batch-Betrieb möglich und somit hinsichtlich der großtechnischen Realisierbarkeit und der Wirtschaftlichkeit weniger interessant.

Im allgemeinen sind die zu oxidierenden Alkinalkohole (Alkinole) Verbindungen, die mindestens eine einbindige Gruppierung der Formel -CH2-OH und mindestens eine zweibindige Gruppierung der Formel -C≡C- enthalten.

Bevorzugt handelt es sich bei den zu oxidierenden Alkinalkoholen um lineare oder verzweigte primäre Alkohole mit 3-30 C-Atomen, um zyklische Alkohole mit 8-30 C-Atomen oder um durch einen aromatischen Rest substituierte Alkohole mit 6-30 C-Atomen, die mindestens eine Gruppe der Formel -C≡C- enthalten,
wobei ein Wasserstoff oder mehrere Wasserstoffe unabhängig voneinander durch F, Cl, Br, I, NO₂ ,ONO, CN, NC oder SCN ersetzt sein können,
oder wobei eine CH₂-Gruppe oder mehrere CH₂-Gruppen unabhängig voneinander durch O, NH, C=O, CO₂, S, S=O, SO₂, P=O oder PO₂ ersetzt sein können,
oder eine CH-Gruppe oder mehrere CH-Gruppen unabhängig voneinander durch N, B oder P ersetzt sein können, oder quartäre Kohlenstoffe durch Si, Sn oder Pb ersetzt sein können.

Besonders bevorzugt sind Alkinalkohole R¹-C≡C-CH₂OH, R¹-C≡C-CH₂-CH₂OH oder R¹-C≡C-CH₂-CH₂-CH₂OH, R¹-O-CR²R³-C≡C-CH₂OH, R¹-O-CR²R³-C≡C-CH₂-CH₂OH oder R¹-O-CR²R³-C≡C-CH₂-CH₂-CH₂OH
mit R¹ in der Bedeutung H, Methyl, Ethyl oder eines linearen oder verzweigten C₃-C₁₂-Rests, insbesondere eines n-Propyl-, Isopropyl-, 1-oder-2-n-Butyl-, 2-Methylpropyl-, 1-, 2- oder 3-n-Pentyl-, 2- oder 3-Methyl-1-butyl-, 1,2-Dimethylpropyl-, tert-butyl-, neopentyl- oder tert-pentylrests,
oder eines gesättigten oder ungesättigten cyclischen C₃-C₁₂-Rests, insbesondere eines Cyclopropyl-, Cyclobutyl-, Cyclopentyl-, Cyclopentenyl-, Methylcyclopentyl-, Methylcyclopentenyl-, Cyclohexyl-, Cyclohexenyl-, Methylcyclohexyl-, Methylcyclohexenyl-, Cycloheptyl-, Cyclooctyl-, Cyclododecyl- oder Decalinylrests
oder eines C₆-C₁₂-Aryl- oder Aralkylrests, insbesondere eines Phenyl-, Benzyl-, Phenethyl-, Naphthyl-, Biphenylyl-, Anthryl-, Phenanthryl-, Azulenyl-, Anthrachinonyl-, 2-, 3- oder 4-Methylphenyl-, 2,3-, 2,4 oder 2,5-Dimethylphenyl- oder Mesitylylrests,
oder eines C₆-C₁₂-Heteroaryl- oder Heteroaralkylrests, insbesondere eines Furyl-, Pyrrolyl-, Thienyl-, Benzofuranyl-, Isobenzofuranyl-, Benzothiyl-, Isobenzothienyl-, Indolyl-, Isoindolyl-, Indolizinyl-, Pyrazolyl-, Imidazolyl-, Oxazolyl-, Thiazolyl-, Isoxazolyl-, Isothiazolyl-, Indazolyl-, Carbazolyl-, Benzotriazolyl-, Purinyl-, Pyridyl-, Pyridazinyl-, Pyrimidyl-, Pyrazinyl-, Chinolinyl-, Isochinolinyl-, Chinoxalinyl-, Chinazolinyl-, Cinnolinyl-, Phenanthridinyl-, Acridinyl-, 1,10-Phenanthrolinyl-, Phenazinyl-, Phenothiazinyl- oder Phenoxazinylrests.
oder in der Bedeutung R⁴R⁵R⁶Si mit R⁴, R⁵ und R⁶ unabhängig voneinander in der Bedeutung C₁-C₁₂-Alkyl, insbesondere Methyl, Ethyl, n-Propyl, Isopropyl oder n-Butyl,
oder C₁-C₁₂-Oxyalkyl, insbesondere Methoxy, Ethoxy, n-Propoxy, Isopropoxy oder Butoxy,
C₆-C₁₂-Aryl oder C₇-C₁₂-Aralkyl, insbesondere Phenyl oder Benzyl,
und R² und R³ unabhängig voneinander in der Bedeutung H, C₁-C₁₂-Alkyl, insbesondere Methyl, Ethyl, n-Propyl oder n-Butyl,
C₆-C₁₂-Aryl oder C₇-C₁₂-Aralkyl, insbesondere Phenyl, 2-,3- oder 4-Methylphenyl oder Benzyl
und Alkinole R⁷-CO-C≡C-CH₂OH, R⁷-CO-C≡C-CH₂-CH₂OH oder R⁷-CO-C≡C-CH₂-CH₂-CH₂OH mit R⁷ in der Bedeutung Methyl, Ethyl oder eines linearen oder verzweigten C₃-C₁₂-Rests, insbesondere eines n-Propyl-, Isopropyl-, 1-oder-2-n-Butyl-, 2-Methylpropyl-, 1-, 2- oder 3-n-Pentyl-, 2- oder 3-Methyl-1-butyl-, 1,2-Dimethylpropyl-, tert-butyl-, neopentyl- oder tert-pentylrests,
oder eines gesättigten oder ungesättigten cyclischen C₃-C₁₂-Rests, insbesondere eines Cyclopropyl-, Cyclobutyl-, Cyclopentyl-, Cyclopentenyl-, Methylcyclopentyl-, Methylcyclopentenyl-, Cyclohexyl-, Cyclohexenyl-, Methylcyclohexyl-, Methylcyclohexenyl-, Cycloheptyl-, Cyclooctyl-, Cyclododecyl- oder Decalinylrests
oder eines C₆-C₁₂-Aryl- oder Aralkylrests, insbesondere eines Phenyl-, Benzyl-, Phenethyl-, Naphthyl-, Biphenylyl-, Anthryl-, Phenanthryl-, Azulenyl-, Anthrachinonyl-, 2-, 3- oder 4-Methylphenyl-, 2,3-, 2,4 oder 2,5-Dimethylphenyl- oder Mesitylylrests,
oder eines C₆-C₁₂-Heteroaryl- oder Heteroaralkylrests, insbesondere eines Furyl-, Pyrrolyl-, Thienyl-, Benzofuranyl-, Isobenzofuranyl-, Benzothiyl-, Isobenzothienyl-, Indolyl-, Isoindolyl-, Indolizinyl-, Pyrazolyl-, Imidazolyl-, Oxazolyl-, Thiazolyl-, Isoxazolyl-, Isothiazolyl-, Indazolyl-, Carbazolyl-, Benzotriazolyl-, Purinyl-, Pyridyl-, Pyridazinyl-, Pyrimidyl-, Pyrazinyl-, Chinolinyl-, Isochinolinyl-, Chinoxalinyl-, Chinazolinyl-, Cinnolinyl-, Phenanthridinyl-, Acridinyl-, 1,10-Phenanthrolinyl-, Phenazinyl-, Phenothiazinyl- oder Phenoxazinylrests.
sowie Cl-CH₂-C≡C-CH₂OH und HO-CH₂-C≡C-C≡C-CH₂OH

Ganz besonders bevorzugt sind 2-Propin-1-ol, But-3-in-1-ol, But-2-in-1-ol, Pent-4-in-1,2-diol, 2-Butin-1,4-diol, 4-Chlor-2-butin-1-ol, 4-Acetoxy-2-butin-1-ol, 4-t-Butyldimethylsiloxy-2-butin-1-ol, 3-Phenyl-2-propin-1-ol, 3-Trimethylsilyl-2-propin-1-ol.

Insbesondere eignen sich 2-Propin-1-ol, 4-Chlor-2-butin-1-ol und 2-Butin-1,4-diol.

Bei der als Oxidationskatalysator eingesetzten Nitroxylverbindung handelt es sich im allgemeinen um eine Di-tert.-alkylnitroxylverbindung.

Bevorzugt handelt es sich um eine Nitroxylverbindung der allgemeinen Formel I 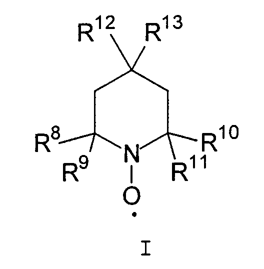 worin die Reste R⁸, R⁹, R¹⁰ und R¹¹ unabhängig voneinander C₁-C₁₂-Alkyl oder C₂-C₁₂-Alkenyl oder C₆-C₁₂-Aryl oder Aralkyl bedeuten,
und die Reste R¹² und R¹³ unabhängig voneinander folgende Bedeutung haben: Wasserstoff, OH, CN, Halogen,
linear oder verzweigt, gesättigt oder ungesättigt C₁-C₂₀-Alkyl-, C₆-C₂₀-Aryl, C₆-C₂₀-Hetaryl oder C₆-C₂₀-Aralkyl, OR¹⁴, O-COR¹⁴, O-COOR¹⁴, OCONHR¹⁴, COOH, COR¹⁴, COOR¹⁴, CONHR¹⁴
wobei R¹⁴ die Bedeutung eines linearen oder verzweigten, gesättigten oder ungesättigten C₁-C₂₀-Alkylrestes, oder die Bedeutung eines C₆-C₂₀-Aryl, C₃-C₂₀-Hetaryl oder C₆-C₂₀-Aralkylrests hat,
-(O-CH₂-CH₂)ₙ-OR¹⁵ , -(O-C₃H₆)ₙ-OR¹⁵, -(O-(CH₂)₄)ₙ-OR¹⁵, -O-CH₂-CHOH-CH₂- (O-CH₂-CH₂-)ₙ-OR¹⁵
mit R¹⁵ in der Bedeutung Wasserstoff, C₁-C₂₀-Alkyl, C₆-C₂₀-Aralkyl, mit n = 1 bis 100 , oder CH₂-CHOH-CH₃ oder CH₂-CHOH-CH₂-CH₃,
NR¹⁶R¹⁷, NHCOR¹⁶, NHCOOR¹⁶, NHCONHR¹⁶,
mit R¹⁶ und R¹⁷ unabhängig voneinander in der Bedeutung eines linearen oder verzweigten, gesättigten oder ungesättigten C₁-C₂₀-Alkylrestes, eines C₆-C₁₂-Cycloalkylrests, oder eines C₆-C₂₀-Aryl , C₃-C₂₀ Hetaryl oder C₆-C₂₀-Aralkylrests,
wobei die Reste R¹² und R¹³ auch zu einem Ring verknüpft sein können,
und wobei die Reste R¹² und R¹³ ihrerseits auch mit COOH, OH, SO₃H, CN, Halogen, prim., sek., tert. Amino oder quart. Ammonium substituiert sein können,
oder die Reste R¹² und R¹³ zusammen auch die Bedeutung =O, =NR¹⁸, =N-OR¹⁸, =N-N=CR¹⁸R¹⁹ mit R¹⁸ und R¹⁹ unabhängig in der Bedeutung Wasserstoff, C₁-C₂₀-Alkyl oder C₆-C₂₀-Aralkyl haben können.

Weiterhin bevorzugt handelt es sich bei der Nitroxylverbindung um zwei Moleküle der Formel I, die über eine Brücke =N-N= in 4-Position verknüpft sind.

Weiterhin bevorzugt handelt es sich bei der Nitroxylverbindung um zwei oder mehrere Moleküle der allgemeinen Formel I, die über einen der beiden Reste R¹² oder R¹³ miteinander verbunden sind. Dabei eignet sich als verknüpfender Rest besonders bevorzugt um O-Alkyl-O, O-CH₂-Aryl-CH₂-O, oder eine Brücke der allgemeinen Formel (O-(CH₂)ₙ-O)ₘ mit n = 2 bis 4 und m = 2 bis 50, insbesondere m = 2 bis 20.

In einer weiteren Ausführungsform handelt es sich bei der Nitroxylverbindung um eine polymere Struktur enthaltend Verbindungen der Formel I, die über die Reste R¹¹ oder R¹² oder R¹¹ und R¹² verknüpft ist.

Dem Fachmann sind eine Vielzahl solcher Verbindungen aus dem Stand der Technik bekannt (EP 1103537, Cirriminna et al., Chem. Commun. 2000, 1441; Bolm et al., Chem. Commun. 1999, 1795; Bobbitt et al., Chem.Commun. 1996, 2745, Miyazawa und Endo, J. Molec. Catal. 49, 1988, L31; M. J. Verhoef et al. in "Studies in Surface Science and Catalysis", Vol. 125, S. 465 ff; D. Brunel et al. in "Studies in Surface Science and Catalysis", Vol. 125, S. 237 ff; Miyazawa und Endo, J. Polymer Sci., Polym. Chem. Ed. 23, 1985, 1527 und 2487; T. Osa, Chem. Lett. 1988, 1423).

Insbesondere eignen sich PIPO (polyamine immobilised piperidinyl oxyl), SiO₂-geträgertes TEMPO, Polystyrol- und Polyacrylsäure-geträgertes TEMPO.

Als Nitroxylverbindung besonders bevorzugt sind Verbindung der allgemeinen Formel I mit R⁸, R⁹, R¹⁰ und R¹¹ in der Bedeutung CH₃
und R¹² und R¹³ unabhängig voneinander in der Bedeutung Wasserstoff, OH, OR¹⁴, O-COR¹⁴, O-COOR¹⁴, OCONHR¹⁴,
wobei R¹⁴ die Bedeutung eines linearen oder verzweigten, gesättigten oder ungesättigten C₁-C₂₀-Alkylrestes, oder die Bedeutung eines C₆-C₂₀-Aryl oder C₆-C₂₀-Aralkylrests hat,
-(O-CH₂-CH₂)ₙ-OR¹⁵, -(O-C₃H₆)ₙ-OR¹⁵, -(O-(CH₂)₄)ₙ-OR¹⁵, -O-CH₂-CHOH-CH₂-(O-CH₂-CH₂-)ₙ-OR¹⁵
mit R¹⁵ in der Bedeutung Wasserstoff, C₁-C₁₀-Alkyl oder C₆-C₁₀-Aralkyl, mit n = 1 bis 100, oder CH₂-CHOH-CH₃ oder CH₂-CHOH-CH₂-CH₃,
NR¹⁶R¹⁷, NHCOR¹⁷, NHCOOR¹⁷, NHCONHR¹⁷,
mit R¹⁶ und R¹⁷ unabhängig voneinander in der Bedeutung Wasserstoff, eines linearen oder verzweigten, gesättigten oder ungesättigten C₁-C₂₀-Alkylrestes, eines C₆-C₁₂-Cycloalkylrests oder eines C₆-C₂₀-Aryl oder C₆-C₂₀-Aralkylrests.

Als Nitroxylverbindung besonders bevorzugt sind weiterhin Verbindungen der allgemeinen Formel I mit R⁸, R⁹, R¹⁰ und R¹¹ in der Bedeutung CH₃
worin R¹² und R¹³ gemeinsam Ketal-Gruppierungen der Formeln O-CHR²⁰CHR²¹-O oder O-CH₂CR²²R²³-CH₂-O mit R²⁰, R²¹, R²² und R²³ unabhängig voneinander in der Bedeutung Wasserstoff oder C₁-C₃-Alkyl bilden,
oder worin die Reste R¹² und R¹³ zusammen die Bedeutung =O haben.

Als Nitroxylverbindung insbesondere bevorzugt sind eine Verbindung der allgemeinen Formel I mit R⁸, R⁹, R¹⁰ und R¹¹ in der Bedeutung CH₃
worin R¹² die Bedeutung Wasserstoff und R¹³ die Bedeutung Wasserstoff, OH, OR¹⁴, O-COR¹⁴,
wobei R¹⁴ die Bedeutung eines linearen oder verzweigten gesättigten C₁-C₁₂-Alkylrestes, oder die Bedeutung eines Aryl- oder Benzylrests hat,
- (O-CH₂-CH₂)ₙ-OR¹⁵, -(O-C₃H₆)ₙ-OR¹⁵, -(O-(CH₂)₄)ₙ-OR¹⁵, -O-CH₂-CHOH-CH₂-(O-CH₂-CH₂-)ₙ-OR¹⁵ mit n = 1 bis 50
und R¹⁵ in der Bedeutung Wasserstoff oder CH₂-CHOH-CH₃ oder CH₂-CHOH-CH₂-CH₃

NR¹⁶R¹⁷, NHCOR¹⁷, mit R¹⁶ und R¹⁷ unabhängig voneinander in der Bedeutung eines linearen oder verzweigten gesättigten C₁-C₁₂-Alkylrestes, oder eines Aryl- oder Benzylrests.

Beispiele für besonders bevorzugt einsetzbare Nitroxylverbindungen sind TEMPO, 4-Hydroxy-TEMPO, 4-Oxo-TEMPO, 4-Amino-TEMPO, 4-Acetamido-TEMPO, 4-Benzoyloxy-TEMPO, 4-Acetoxy-TEMPO und PIPO.

Die Nitroxylverbindung wird im allgemeinen in Mengen von 0,01 bis 50 Mol %, bevorzugt in Mengen von 0,1 bis 20 Mol %, besonders bevorzugt in Mengen von 1 bis 10 Mol % bezogen auf die Menge an zu oxidierendem Alkinalkohol eingesetzt.

Sie kann in der den Alkinalkohol enthaltenden Reaktionskomponente oder in der wässrigen Phase gelöst oder in geträgerter Form als eigenständige Phase eingesetzt werden.

Dem Fachmann sind aus dem Stand der Technik geeignete Hypohalogenite und Hypohalogenit-Zubereitungen bekannt (Ullmann Encyclopedia, 6^{th} Edition, 2001 electronic release; "Chlorine oxides and Chlorine oxygen acids 2. - 4.").

Als Oxidationsmittel wird bevorzugt eine Verbindung eingesetzt, ausgewählt aus der Gruppe der Hypohalogenite, insbesondere Hypochlorit, Hypobromit und Hypoiodit oder deren Gemische. Besonders bevorzugtes Oxidationsmittel ist Hypochlorit. Als Gegenionen sind Wasserstoff, Natrium, Kalium, Kalzium oder Tetraalkylammonium bevorzugt.

In einer besonders bevorzugten Ausführungsform werden technische Hypohalogenitlösungen, insbesondere technische Hypochloritlösungen eingesetzt.

Das verwendete Oxidationsmittel kann auch in situ, insbesondere elektrochemisch, durch Hydrolyse, insbesondere durch Hydrolyse von N-Chlorverbindungen, oder durch Redoxreaktionen, wie bei Hypochlorit- oder Hypobromitlösungen durch Disproportionierung von Chlor bzw. Brom in alkalisch-wässriger Lösung, oder durch die Redoxreaktion zwischen Hypochlorit und Bromid, die zur Bildung von Hypobromit führt, erzeugt werden.

Die verwendeten Oxidationsmittel, insbesondere Hypochlorit, Hypobromit und Hypoiodit werden vorzugsweise in Konzentrationen von 0,1 M bis zu ihrer jeweiligen Sättigungskonzentration als wässrige Lösungen eingesetzt.

Der pH-Wert der wässrigen Lösungen des Oxidationsmittels liegt im allgemeinen zwischen 7 und 14, bevorzugt zwischen 7 und 11, besonders bevorzugt zwischen pH 8 und 10. Die Einstellung des gewünschten pH-Werts erfolgt im allgemeinen durch Zugabe einer Säure, bevorzugt Schwefelsäure, Kohlendioxid, Essigsäure, Ameisensäure, Salzsäure oder Phosphorsäure, besonders bevorzugt Kohlendioxid oder Essigsäure, oder einer Base, bevorzugt Natriumhydroxid und Kaliumhydroxid, besonders bevorzugt Natriumhydroxid. Auch kann die Einstellung des pH-Werts durch Zugabe eines Puffers, bevorzugt Natriumhydrogencarbonat oder Natriumdihydrogenphosphat, besonders bevorzugt Natriumhydrogencarbonat erfolgen.

Weitere mögliche Zusätze sind Salze, z.B. Alkali-, Erdalkalioder Ammoniumhalogenide, -carbonate oder -sulfate.

Der pH-Wert der wässrigen Phase der Reaktionsmischung liegt im allgemeinen kleiner pH 7, bevorzugt zwischen pH 1 und 7, besonders bevorzugt zwischen pH 3 und 6.

Die Einstellung des gewünschten pH-Werts der Reaktionsmischung erfolgt im allgemeinen durch Zugabe einer Säure, bevorzugt Schwefelsäure, Kohlendioxid, Essigsäure, Ameisensäure, Salzsäure oder Phosphorsäure, besonders bevorzugt Schwefelsäure, Salzsäure, Essigsäure oder Phosphorsäure oder einer Base, bevorzugt Natriumhydroxid, Kaliumhydroxid, Natriumcarbonat und Natriumhydrogencarbonat, besonders bevorzugt Natriumhydroxid. Sie kann ebenfalls durch Zugabe eines Puffers, bevorzugt Natriumhydrogencarbonat oder Natriumdihydrogenphosphat, besonders bevorzugt Natriumhydrogencarbonat erfolgen.

Bei der Darstellung von Carbonsäuren mit hoher Säurestärke, wie beispielsweise Propiolsäure oder Acetylendicarbonsäure wird der pH-Wert der Reaktionsmischung durch die entstehende Säure auf unter 7 gehalten.

Die Reaktionstemperatur beträgt im allgemeinen -10 bis +80°C, bevorzugt -5 bis +30°C, besonders bevorzugt -5 bis +15°C.

Die Verfahren werden bevorzugt bei Normaldruck durchgeführt.

Alle vorstehenden Symbole der vorstehenden Formeln weisen ihre Bedeutung jeweils unabhängig voneinander auf.

Die folgenden Beispiele dienen der weiteren Erläuterung der Erfindung.

### Vergleichsbeispiel 1 : Oxidation von 2-Propin-1-ol bei pH >7

7,5 g (134 mmol) 2-Propin-1-ol werden zusammen mit 1,2 g (7 mmol) 4-Hydroxy-TEMPO in 10 ml Wasser gelöst und auf 5°C gekühlt.
Unter intensiver Rührung werden 800 g 2,2 M Natriumhypochloritlösung (technische Bleichlauge; zuvor mit gasförmigen CO₂ auf pH 9,5 eingestellt) so zugegeben, dass die Innentemperatur nicht über 10 °C steigt. Der pH der Reaktionsmischung wird durch Zusatz von Natronlauge bei >8 gehalten.
Nach vollständiger Zugabe wird noch 1 Stunde bei max. 10°C nachgerührt. Eine Analyse der Reaktionsmischung zeigt, dass ca. 40% des eingesetzten Alkohols nicht umgesetzt wurden und die Reaktionsmischung praktisch keine Propiolsäure enthält.

### Beispiel 1 : Oxidation von 2-Propin-1-ol zu Propiolsäure

75 g (1340 mmol) 2-Propin-1-ol werden zusammen mit 11,5 g (67 mmol) 4-Hydroxy-TEMPO in 75 ml Wasser gelöst und auf 5°C gekühlt.
Unter intensiver Rührung werden 1563 g 2,2 M Natriumhypochloritlösung (ca. 1,2 1 technische Bleichlauge; zuvor mit gasförmigem CO₂ auf pH 9,5 eingestellt) so zugegeben, dass die Innentemperatur nicht über 10 °C und zugleich der pH-Wert nicht über 7 steigt. Insbesondere die ersten 20% der Bleichlauge müssen daher langsam zugegeben werden.
Nach vollständiger Zugabe wird mit insgesamt 600 ml Methyl-t-butylether (MTBE) extrahiert (dabei fallen kleinere Mengen an Propiolsäurepropargylester an). Nach Abtrennung der organischen Phasen wird die wässrige Phase mit ca. 64 g konzentrierter Schwefelsäure auf pH 0 eingestellt und 3-mal mit je 300 ml MTBE extrahiert. Die wässrige Phase wird verworfen.
Die MTBE-Phasen werden vereint, getrocknet und ergeben nach teilweiser destillativer Entfernung des MTBE eine ca. 50%ige Lösung von Propiolsäure in MTBE, die 41 g (590 mmol) Propiolsäure entsprechend 44% Ausbeute mit einer Reinheit von 90-92% enthält. Diese Lösung kann direkt zur Darstellung von z. B. Propiolsäureestern verwendet werden.

### Beispiel 2 : Kontinuierliche Oxidation von 2-Propin-1-ol zu Propiolsäure

112 g (2,0 mol) 2-Propin-1-ol werden zusammen mit 17,2 g (100 mmol) 4-Hydroxy-TEMPO in 112 ml Wasser gelöst (Lösung A).
3 kg 2,2 M Natriumhypochloritlösung (technische Bleichlauge) werden mit Essigsäure auf pH 9,5 eingestellt (Lösung B).
Unter intensiver Rührung werden Lösung A mit 1,11 g/min (entsprechend 9,3 mmol Propargylalkohol / min) und Lösung B mit 13,9 g/min (entsprechend 25 mmol Hypochlorit / min) in einen 1-Liter-Rührkessel gefördert, aus dem zugleich kontinuierlich die Reaktionsmischung so abgezogen wird, dass das Volumen der Reaktionsmischung bei etwa 600 ml konstant gehalten wird (entsprechend einer mittleren Verweilzeit von ca. 40 min). Dabei wird durch Kühlung die Temperatur zwischen 5 und 10 °C gehalten. Der pH-Wert der Reaktionsmischung liegt zwischen 5 und 6.

Die kontinuierlich abgenommene Reaktionsmischung wird analog Beispiel 3 mit MTBE gewaschen, mit Schwefelsäure auf pH 0 gestellt und das Reaktionsprodukt mit MTBE aus der wässrigen Phase isoliert. Die so erhaltenen Ausbeuten an Propiolsäure liegen bei 50-60%.

### Beispiel 3 : Zweiphasige Oxidation von 2-Propin-1-ol zu Propiolsäurepropargylester bzw. Propiolsäure

Eine Lösung von 10 g (180 mmol) 2-Propin-1-ol in 30 ml Methylenchlorid wird mit 1,55 g (9 mmol) 4-Hydroxy-TEMPO versetzt und auf 5°C gekühlt.
Unter intensiver Rührung werden 207 g 2,2 M Natriumhypochloritlösung (technische Bleichlauge; zuvor mit gasförmigem CO₂ auf pH 9,5 eingestellt) so zugegeben, dass die Innentemperatur nicht über 10 °C und der pH-Wert nicht über 7 steigt.
Nach vollständiger Zugabe ergibt sich ein pH-Wert von 3,3. Organische und wässrige saure Phase werden getrennt. Die organische Phase enthält 1,7 g Propiolsäurepropargylester. Diese wird unter Rühren mit 50 ml 1 N NaOH bei 35°C versetzt und nach 1 h abgetrennt. Die alkalische wässrige Phase wird mehrfach mit Methyl-t-butylether (MTBE) extrahiert. Die vereinigten MTBE-Phasen enthalten nicht umgesetzten bzw. aus der Esterspaltung gewonnenen Propargylalkohol, der wieder eingesetzt werden kann.
Die wässrigen sauren und alkalischen Phasen werden ebenfalls vereint, mit konzentrierter Schwefelsäure auf pH 0 eingestellt und 3-mal mit je 100 ml MTBE extrahiert. Die wässrige Phase wird verworfen.
Die MTBE-Phasen werden vereint, getrocknet und ergeben nach teilweiser destillativer Entfernung des MTBE eine 50%ige Lösung von Propiolsäure in MTBE, die 9,0 g (129 mmol) Propiolsäure entsprechend 71% Ausbeute enthalten. Diese Lösung kann direkt zur Darstellung von z. B. Propiolsäureestern verwendet werden.

### Beispiel 4 : Oxidation von 3-Butin-1-ol zu Acetylenessigsäure

91 g (1,3 mol) 3-Butin-1-ol werden zusammen mit 11,5 g (67 mmol) 4-Hydroxy-TEMPO in 75 ml Wasser gelöst und auf 5°C gekühlt.
Unter intensiver Rührung werden 1563 g 2,2 M Natriumhypochloritlösung (technische Bleichlauge; zuvor mit gasförmigem CO₂ auf pH 9,5 eingestellt) so zugegeben, dass die Innentemperatur nicht über 10 °C und zugleich der pH-Wert nicht über 7 steigt. Nach vollständiger Zugabe wird mit Natronlauge auf pH 7 gestellt und mit insgesamt 500 ml Methyl-t-butylether (MTBE) extrahiert. Nach Abtrennung der organischen Phasen wird die wässrige Phase mit konzentrierter Schwefelsäure auf pH 1 eingestellt und 2 mal mit je 300 ml MTBE extrahiert. Die wässrige Phase wird verworfen.
Die MTBE-Phasen werden vereint, getrocknet und ergeben nach destillativer Entfernung des MTBE Acetylenessigsäure in 41% Ausbeute.

### Beispiel 5: Oxidation von 2-Butin-1,4-diol zu Acetylendicarbonsäure

Eine Lösung von 4,76 g (55.3 mmol) 2-Butin-1,4-diol in 50 ml Wasser wird mit 0,63 g (3,66 mmol) 4-Hydroxy-TEMPO versetzt, die Mischung auf 3°C gekühlt und unter Rühren und Kühlen bei Temp. von max. 10°C mit 111 ml 1,8 M Natriumhypochloritlösung (technische Bleichlauge; zuvor mit gasförmigem CO₂ auf pH 9,5 eingestellt) innerhalb 1 Std. versetzt. Man lässt noch 2 Std. weiterreagieren, schüttelt dann den Ansatz mit 50 ml MTBE, säuert die wässrige Phase mit konzentrierter Schwefelsäure auf pH 0 an und extrahiert die Wasserphase zweimal mit je 50 ml MTBE. Durch Eindampfen der beiden MTBE-Extrakte im Vakuum werden 6,3 g (50 %) Acetylendicarbonsäure in Form von farblosen Kristallen erhalten.

## Patentansprüche

1. Verfahren zur Herstellung von Alkincarbonsäuren, **gekennzeichnet durch** die Oxidation
eines Alkinalkohols
mit 1 bis 10 mol-Äquivalenten eines Hypohalogenits bezogen auf die Anzahl zu oxidierender funktionaler Gruppen
in Gegenwart einer Nitroxylverbindung
bei einem pH-Wert kleiner 7.

2. Verfahren zur Herstellung von Alkincarbonsäure-Alkinalkoholestern, **gekennzeichnet durch**
die teilweise Oxidation des Alkinalkohols
zu Alkincarbonsäure-Alkinalkoholestern
mit 0,5 bis 5 mol-Äquivalenten eines Hypohalogenits bezogen auf die Anzahl zu oxidierender funktionaler Gruppen
in Gegenwart einer Nitroxylverbindung
bei einem pH-Wert kleiner 7.

3. Verfahren nach einem der Ansprüche 1 oder 2, **dadurch gekennzeichnet, daß** eine wässrige Phase verwendet wird.

4. Verfahren nach Anspruch 3, **dadurch gekennzeichnet, daß** die wässrige Phase ein oder mehrere mit Wasser mischbare Lösungsmittel enthält.

5. Verfahren nach einem der Ansprüche 1 und 2, **dadurch gekennzeichnet, daß** die Reaktion in einem Mehrphasensystem durchgeführt wird.

6. Verfahren nach Anspruch 5, **dadurch gekennzeichnet, daß** mindestens eine wässrige und eine organische Phase verwendet wird.
